# EUROPÄISCHE PATENTANMELDUNG

(11) **EP 1 078 669 A1**
(43) Veröffentlichungstag der Anmeldung: **28.02.2001**
(21) Anmeldenummer: 99810752.8
(22) Anmeldetag: 23.08.1999
(51) Int. Cl.: B01D 9/00

(54) **Verfahren und Kristallisator zur Reinigung von Stoffen oder Stoffgemischen**

(71) Anmelder: Sulzer Chemtech AG, 8404 Winterthur (CH)
(72) Erfinder: Ulrich, Joachim Prof. Dr., 06120 Halle/Saale (DE); Peters-Erjawetz, Sandra Dipl.-Ing., 28197 Bremen (DE); Cellucci, Silvia, 7303 Mastils (CH); Stadler, Ralph, 9470 Buchs (CH)
(74) Vertreter: Hasler, Erich, Dr.

(57) **Zusammenfassung**

Die vorliegende Erfindung betrifft ein Verfahren und eine Vorrichtung zur Trennung oder Reinigung von gelösten, geschmolzenen oder flüssigen Verbindungen oder Verbindungsgemischen mittels fraktionierter Schmelzkristallisation, bei welchem Verfahren eine zu reinigende Verbindung oder ein zu trennendes Verbindungsgemisch in einen Behälter (13) eingebracht und in Kontakt mit Wärmetauscherflächen (15) gebracht wird, die Wärmetauscherflächen (15) auf der einen Seite mittels eines Wärmetransfermediums von einer Temperatur, welche vorzugsweise höher ist als jene, bei welcher die gewünschte Verbindung kristallisiert, auf eine Endtemperatur, bei welcher die gewünschte Verbindung wenigstens teilweise kristallisiert, abgekühlt werden, sodass sich eine aus Kristallen bestehende Kristallschicht der gewünschten Verbindung auf der anderen Seite der Wärmetauscherflächen bildet, dann der nicht-kristallisierte Teil der Verbindung oder des Verbindungsgemisches abgelassen wird und die Kristallschicht geschmolzen und die Schmelze in wenigstens einer Fraktion gesammelt und gegebenfalls, falls der gewünschte Reinheitsgrad noch nicht erreicht ist, einem neuen Reinigungszyklus unterworfen wird. Zum Schwitzen der Kristallschicht wird wenigstens zeitweise ein Gasstrom eines gasförmigen Wärmetransfermediums über die Kristallschicht geleitet wird, wobei der Gasstrom vorgängig auf eine Temperatur erwärmt wird, welche höher als die Schmelztemperatur der Kristallschicht ist.

## Beschreibung

Die Erfindung betrifft ein Verfahren gemäss Oberbegriff von Anspruch 1 und einen Kristallisator gemäss Oberbegriff von Anspruch 11 zur Trennung oder Reinigung von gelösten, geschmolzenen oder flüssigen Verbindungen oder Verbindungsgemischen.

Die US 3,621,664 (Saxer), deren Offenbarungsgehalt hiermit unter Bezugnahme aufgenommen wird, offenbart einen Kristallisator bestehend aus einem in einem Behälter angeordneten Rohrbündel. Der Kristallisator wird zur Reinigung von verunreinigten Verbindungen resp. der Trennung von Stoffgemischen eingesetzt. Bei der Reinigung wird eine Schmelze des zu reinigenden Gemisches als Fallfilm oder als volle Rohrfüllung durch die Rohre geleitet. Zur Abscheidung der gewünschten Verbindung als auf der Innenseite der Rohre anhaftende Kristallschicht werden die Rohre von aussen mittels eines Wärmetransfermediums gekühlt, welches ebenfalls als Rieselfilm an der Aussenseite der Rohre herunterfliessen kann. Die beim Abkühlen gebildete Kristallschicht enthält die gewünschte Verbindung in höherer Reinheit als die Schmelze. Die Kristallschicht wird sodann geschwitzt und abgeschmolzen, indem das auf der Aussenseite der Rohre vorbeiströmende Wärmetransfermedium kontinuierlich oder in diskreten Schritten erwärmt wird. Die Erwärmung der Kristallschicht erfolgt dabei ausschliesslich durch die Wärmetauscherflächen hindurch. Die Erwärmung der Kristallschicht erfolgt derart, dass verschiedene Fraktionen gewonnen werden können, nämlich eine sogenannte "Schwitzphase", welche beim Schwitzen der Kristalle anfällt, und einer "Kristallphase", welche der Kristallschicht nach dem Schwitzen entspricht.

Die beim Reinigungsverfahren anfallenden Fraktionen, d.h. der nicht-kristallisierte Rückstand oder Mutterlauge, die Schwitz- und die Kristallphase, können sodann einem oder mehreren weiteren Reinigungszyklen unterworfen werden, bis die Verbindung in der gewünschten Reinheit vorliegt resp. die Verunreinigungen in der Mutterlauge stark angereichert sind.

Ein Einsatzgebiet des oben beschriebenen Reinigungsverfahrens ist die grosstechnische Reinigung von Ausgangsstoffen, wie substituierte und unsubstituierte Naphthaline, Acrylsäure etc. Aufgrund der riesigen, jährlich zu reinigenden Mengen ist die Wirtschaftlichkeit der eingesetzten Verfahren von grösster Bedeutung. Dabei spielt der Durchsatz und die eingesetzte Energie eine entscheidende Rolle.

Das oben beschriebene Kristallisationsverfahren lässt sich auch mittels eines statischen Kristallisators durchführen. Der statische Kristallisator besteht aus einem Behälter, in welchem eine Anzahl von meist flachen Wärmetauscherflächen angeordnet sind. Diese Wärmetauscherflächen können aus doppelwandigen Kühlelementen bestehen, welche von einem Wärmetransfermedium durchströmt werden. Das sog. statische Kristallisationsverfahren unterscheidet sich vom eingangs beschriebenen dynamischen Verfahren darin, dass die Tankfüllung beim Kristallisationsvorgang "statisch" verharrt, d.h. nicht zirkuliert oder gerührt wird. Entsprechend ist das statische Kristallisationsverfahren bedeutend langsamer als das bei einem Fallfilmkristallisator oder einem Kristallisator mit voller Rohrfüllung angewandte dynamische Kristallisationsverfahren.

Aufgabe der vorliegenden Erfindung ist es, die bekannten Verfahren zur Reinigung von Verbindungen resp. Verbindungsgemischen mittels fraktionierter Kristallisation zu verbessern. Ein weiteres Ziel ist eine bessere Trennleistung bei der fraktionierten Kristallisation zu erzielen. Zudem ist es ein Ziel, das Verfahren wirtschaftlicher zu gestalten. Eine weitere Aufgabe der vorliegenden Erfindung ist es, eine Vorrichtung zur Durchführung des Verfahrens zur Verfügung zu stellen.

Erfindungsgemäss wird dies durch ein Verfahren realisiert, bei welchem
a) ein zu reinigendes Verbindungsgemisch in einen Behälter eingebracht und in Kontakt mit Wärmetauscherflächen gebracht wird,
b) die Wärmetauscherflächen auf der einen Seite mittels eines Wärmetransfermediums von einer Temperatur, welche vorzugsweise höher ist als jene, bei welcher die gewünschte Verbindung kristallisiert, auf eine Endtemperatur, bei welcher die gewünschte Verbindung wenigstens teilweise kristallisiert, abgekühlt werden, sodass sich eine aus Kristallen bestehende Kristallschicht der gewünschten Verbindung auf der anderen Seite der Wärmetauscherflächen bildet,
c) dann der nicht-kristallisierte Teil des Verbindungsgemisches abgelassen wird und
d) die Kristallschicht geschmolzen und die Schmelze in wenigstens einer Fraktion gesammelt und gegebenfalls, falls der gewünschte Reinheitsgrad noch nicht erreicht ist, einem neuen Reinigungszyklus mit den Schritten a) bis d) unterworfen wird, und
e) zum Schmelzen wenigstens zeitweise ein Gasstrom eines gasförmigen Wärmetransfermediums über die Kristallschicht geleitet wird, wobei der Gasstrom vorgängig auf eine Temperatur erwärmt wird, welche höher als die Schmelztemperatur der Kristallschicht ist.

Dieses Verfahren, nachfolgend auch als Gasschwitzen bezeichnet, hat den überraschenden Vorteil, dass durch den Gasstrom die Trennleistung verbessert wird. Obwohl die genauen Ursachen nicht völlig geklärt sind, wird angenommen, dass durch das gasförmige Wärmetransfermedium der Stoffaustausch zwischen der Kristall- und der Flüssigphase verstärkt wird, was eine bessere Trennung im Vergleich zu Verfahren, bei welchen kein gasförmiges Wärmetransfermedium über die Kristallschicht geleitet wird, zur Folge hat. Da die Verunreinigungen vornehmlich an der Oberfläche der Kristalle angereichert sind, lassen sich die Verunreinigungen beim Schwitzen mit einem gasförmigen Medium bevorzugt abschmelzen. Beim Gasschwitzen können die Poren geöffnet werden, wobei die Viskosität der anhaftenden Schmelze herabgesetzt wird, die Kristallschicht im übrigen aber stabil bleibt. Die von oben nach unten fliessende Schwitzflüssigkeit wirkt als Waschflüssigkeit für die von der Schwitzflüssigkeit überstrichene Kristallschicht.

Ein weiterer Vorteil des Verfahrens ist, dass durch eine Wärmezufuhr von zwei Seiten (flüssiges Wärmetransfermedium auf der der Kristallschicht abgewandten Seite der Wärmetauscherflächen und gasförmiges Wärmetransfermedium auf der die Kristallschicht aufweisenden Seite der Wärmetauscherflächen) die Geschwindigkeit für das Abschmelzen der gebildeten Kristallschicht erhöht werden kann.

Besonders vorteilhaft ist das Gasschwitzen bei Stoffen oder Stoffgemischen, welche bekanntermassen an den Wärmetauscherflächen schlecht haften. In solchen Fällen können die zu reinigenden Stoffe oder Stoffgemische ausschliesslich durch Gasschwitzen geschwitzt werden, wobei das in den Wärmetauscherflächen zirkulierende resp. an der der Kristallschicht abgewandten Seite der Wärmetauscherflächen herabfliessende Wärmetransfermedium vorzugsweise auf einer Temperatur unterhalb der Schmelztemperatur des zu reinigenden Stoffes resp. Stoffgemisches gehalten wird, sodass eine gute Haftung der Schicht an den Wärmetauscherflächen gewährleistet ist.

Vorteilhaft geschieht das Schmelzen, indem die Kristallschicht zuerst geschwitzt (sog. Schwitzphase) und dann abgeschmolzen wird (sog. Schmelzphase), wobei wenigstens zum Schwitzen wenigstens zeitweise ein Gasstrom eines gasförmigen Wärmetransfermediums über die Kristallschicht geleitet wird. Das Schwitzen der Kristallschicht kann ausschliesslich durch den Einsatz des erwärmten Gasstromes erfolgen. Durch die Zuführung von Wärme auch von der der Kristallschicht abgewandten Seite der Wärmetauscherflächen her (mittels eines flüssigen oder gegebenenfalls gasförmigen Wärmetransfermediums) kann die Schwitzphase verkürzt werden. Vorteilhaft wird zum Schwitzen einerseits die Temperatur des Wärmetransfermediums erhöht und andererseits wenigstens zeitweise ein erwärmter Gasstrom eines gasförmigen Wärmetransfermediums über die Kristallschicht geleitet.

Gemäss einer bevorzugten Ausführungsvariante wird der erwärmte Gasstrom im wesentlichen während des ganzen Schmelzvorgangs, also während des Kristallschwitzens und des Abschmelzens der Kristalle, eingesetzt. Damit kann der Reinigungsprozess bedeutend verkürzt werden und die Wirtschaftlichkeit des Verfahrens verbessert werden.

Obwohl grundsätzlich die unterschiedlichsten Gase eingesetzt werden können, wird vorzugsweise ein inertes oder bezüglich des zu reinigenden Verbindungsgemisches nicht-reaktives Gas eingesetzt. Das Gas kann ein Edelgas, Stickstoff, Luft, Kohlendioxid, Wasserdampf, Freon, aliphatische oder cyclische unsubstituierte Kohlenwasserstoffe, substituierte Halogen-Kohlenwasserstoffe wie z.B. Freone sein.

Obwohl der Gasstrom in Gegenrichtung zur abfliessenden Schmelze über die Kristallschicht geleitet werden kann, wird der Gasstrom gemäss einer bevorzugten Ausführungsvariante in Gleichrichtung zur abfliessenden Schmelze über die Kristallschicht geleitet. Das heisst, der erwärmte Gasstrom wird im Kopfbereich des Kristallisators eingeleitet und im unteren Bereich des Kristallisators wieder abgeführt. Dadurch ergibt sich eine vergleichsweise bessere Trennleistung, als wenn der Gasstrom in Gegenrichtung zur abfliessenden Schmelze über die Kristallschicht geleitet wird.

Vorteilhaft wird der Gasstrom im Kreis geführt. Zweckmässigerweise wird der Gasstrom auf eine Temperatur erhitzt, welche wenigstens 5 °C, vorzugsweise wenigstens 15 °C und ganz besonders bevorzugt wenigsten 25 °C über dem Schmelzpunkt der die Kristallschicht bildenden Kristalle liegt. Da Gase oder Gasgemische einen vergleichsweise geringen Wärmeinhalt haben, ist der Abschmelzvorgang gut kontrollierbar. Durch Gase kann genügend Energie zugeführt werden, sodass einerseits ein spürbare Verbesserung der Trennleistung und andererseits eine signifikante Beschleunigung des Verfahrens erreicht wird. Obwohl die Erwärmung des Gasstromes erfolgen kann, indem eine bestimmte Energiemenge pro Zeiteinheit zugeführt wird, wird gemäss einer bevorzugten Variante der Gasstrom geregelt erwärmt. Dies hat den Vorteil, dass der Reinigungsprozess gut optimierbar ist.

Besonders vorteilhaft wird das Gasschwitzen zur Reinigung von wachsähnlichen Verbindungen oder Verbindungsgemischen, Fettsäuregemischen, Acrylsäure mittels fraktionierter Kristallisation eingesetzt. Dabei wird die Kristallschicht vor dem Abschmelzen geschwitzt, indem wenigstens zeitweise ein Gasstrom eines gasförmigen Wärmetransfermediums über die Kristallschicht geleitet wird, wobei der Gasstrom vorgängig auf eine Temperatur erwärmt wird, welche höher als die Schmelztemperatur der Kristallschicht ist.

Gegenstand der vorliegenden Erfindung ist auch ein Kristallisator gemäss Kennzeichen von Anspruch 11 gekennzeichnet durch wenigstens einen Gaseinlass für den Einlass eines Gases in den Behälterraum und wenigstens einen Gasauslass für den Auslass des gasförmigen Behälterinhalts aus dem Behälterraum, einer Gaszuleitung mit Heizmitteln, welche mit dem Gaseinlass in Verbindung steht zur Erwärmung des zuzuführenden Gases, und Fördermitteln zur Förderung des Gases in den Behälterraum resp. aus diesem heraus. Der erfindungsgemässe Kristallisator hat den Vorteil, dass er ermöglicht, erwärmtes Gas in den Kristallisatorraum einzubringen und abzuführen und so die Trennleistung und den Durchsatz zu erhöhen.

Obwohl das Gas grundsätzlich nur für den Einmalgebrauch eingesetzt werden kann, sind gemäss einer vorteilhaften Ausführungsform der Gasein- und der Gasauslass durch eine Verbindungsleitung miteinander verbunden. Dies erlaubt es, das Gas zu zirkulieren und mehrfach zu gebrauchen. Die Verbindungsleitung kann dabei teilweise durch die Zirkulationsleitung zur Zirkulation der Schmelze gebildet sein, da beim Abschmelzen der Kristallschicht in der Regel keine Zirkulation des sich am Boden gesammelten flüssigen Tankinhalts mehr stattfindet.

Zweckmässigerweise ist die eine Gasanschlusstelle in Abstand zum Kristallisatorboden vorgesehen. Dadurch wird verhindert, dass Flüssigkeit (Schmelze) unbeabsichtigt austreten oder in die Verbindungsleitung für die Zirkulation des Gases eintreten kann. Zu diesem Zweck kann es auch angezeigt sein, die Gasverbindungsleitung mittels Absperrmitteln absperrbar zu machen, sodass diese auch näher beim Boden angeschlossen werden kann.

Obwohl die Heizmittel durch eine elektrisch heizbare Heizfläche oder Heizschlange gebildet sein können, sind diese vorteilhaft als Wärmetauscher, z.B. eine Heizschlange, in welcher ein Wärmetauschermedium zirkulieren kann, ausgebildet. Ein derartiger Wärmetauscher hat den Vorteil, dass dessen Temperatur gut regulierbar ist. Zweckmässigerweise stehen die Heizmittel mit einem Temperaturfühler und einem Regulierventil, welches den Durchfluss des Wärmetauschermediums im Wärmetauscher kontrolliert, in Verbindung. In Verbindung mit einem Steuergerät ist auf diese Weise ein Regelkreis realisiert und der Reinigungsprozess ist dadurch gut optimierbar und, wenn dieser einmal optimiert ist, gut kontrollier- und reproduzierbar. Vorteilhaft sind die Fördermitteln derart ausgebildet, dass im Betrieb das Gas vom Boden- zum Kopfbereich des Kristallisators gefördert wird.

Das erfindungsgemässe Verfahren und der erfindungsgemässe Kristallisator eignen sich insbesondere zur Reinigung nachfolgender Verbindungen: Acrylsäure, Metacrylsäure, Abwasser, Methyldiphenylisozyanat (MDI), Toluoldiisozyanat (TDI), Caprolactam, Benzoesäure, Bisphenol-A-, Nitrochlorbenzol, geradkettige und verzweigte Fettsäuren, Hydrazin, Phenole wie para-, meta und ortho-Kresol, 2.6 und 3,5-Dimethylphenol, Naphthol und o,o-Diphenol, chlorierte Kohlenwasserstoffe wie Dichlorbenzol und Nitrochlorbenzol, Naphthalin, 1- 2-Methylnaphthalin, Acenaphthen, Fluoren, Phenanthren, Adipinsäuredinitril, Hexamethylendiamin, Lactide (Polylactide), Chlorpyrifos sowie Paraffine ab C₁₇. Das Verfahren lässt sich auch für beliebig andere, kristallisierbare Verbindungen anwenden.

Es zeigt
- Figur 1:: schematisch eine erste Ausführungsform eines erfindungsgemässen Kristallisators, teilweise im Aufriss; und
- Figur 2:: schematisch eine zweite Ausführungsform eines erfindungsgemässen Kristallisators, ebenfalls teilweise im Aufriss; und
- Figur 3:: ein Vergleich der Trennleistung des erfindungsgemässen Verfahrens mit dem Stand der Technik.

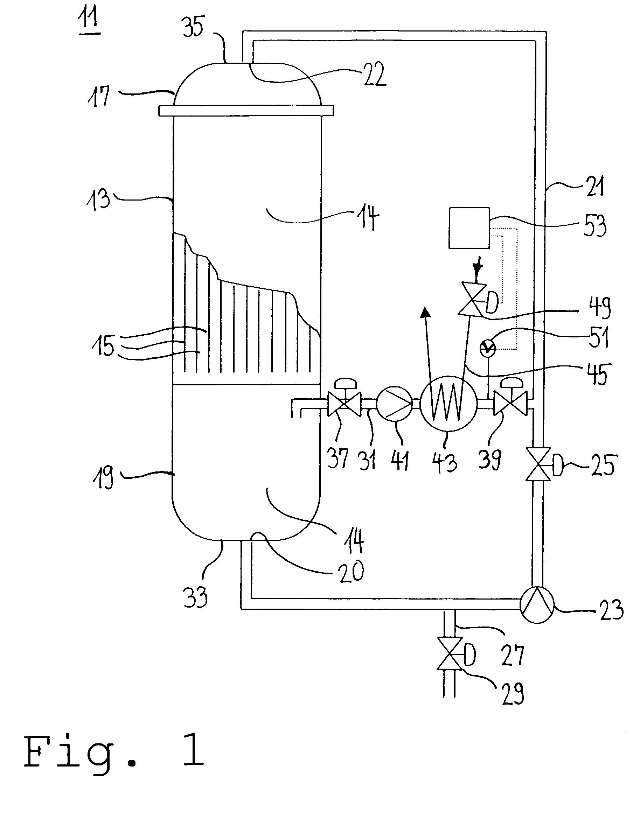

Der Kristallisator 11 gemäss Figur 1 hat einen Behälter 13 mit einem Behälterraum 14, in welchem eine Anzahl von kühl- resp. erwärmbaren Kristallisations- oder Wärmetauscherflächen 15 angeordnet sind. Die Wärmetauscherflächen 15 erstrecken sich koaxial zur Behälterlängsachse vom Kopfbereich 17 des Kristallisators bis zum Bodenbereich 19. Zwecks Vereinfachung der Darstellung sind die Leitungen, Pumpen und Wärmetauscherapparate, welche in Verbindung mit den Wärmetauscherflächen einen Kühl- resp. Wärmekreislauf bilden, nicht eingezeichnet. Der Bodenbereich 19 entspricht ungefähr dem Füllvolumen des Kristallisators 11. Eine Zirkulationsleitung 21 verbindet eine Anschlussstelle 20 im Bodenbereich 19 des Kristallisators mit einer zweiten Anschlussstelle 22 im Kopfbereich 17. Eine Pumpe 23 dient der Förderung des im Betrieb im Bodenbereich 19 sich befindlichen flüssigen Tankinhalts zum Kristallisatorkopfbereich 17, damit die zu reinigende Flüssigkeit oder Mutterlauge als Fall- oder Rieselfilm an den Wärmetauscherflächen nach unten fliessen kann. Mittels eines Ventils 25 ist die Flüssigkeitszirkulationsleitung 21 absperrbar. Eine Leitung 27 mit Absperrventil 29 dient der Zu- resp. Abführung von Roh- und von gereinigtem Material.

Erfindungsgemäss unterscheidet sich der Kristallisator von bekannten Kristallisatoren dadurch, dass Mittel vorgesehen sind, um ein erwärmtes Gas an den Wärmetauscherflächen 15 entlang zu führen. Dies hat den Vorteil, dass der Durchsatz erhöht und die Trennleistung verbessert werden kann, da der Schwitz- und Schmelzvorgang beschleunigt und, überraschenderweise, die Reinigungswirkung verbessert werden kann.

Der Kristallisator 11 gemäss Fig. 1 weist zur Zirkulation eines Gases eine leitungsmässige Verbindung auf, welche den unterhalb der Wärmetauscherflächen liegenden Kristallisatorraum mit demjenigen oberhalb der der Wärmetauscherflächen verbindet, sodass der gasförmige Tankinhalt zirkuliert und ein Gasstrom an den Wärmetauscherflächen vorbei geleitet werden kann.

Die leitungsmässige Verbindung ist gemäss Figur 1 realisiert durch eine Verbindungsleitung 31, welche in Abstand zum Kristallisatorboden 33 den Behälterraum mit der Zirkulationsleitung 21 verbindet. Die Zirkulationsleitung 21, welche in den Kristallisatordeckel 35 mündet, kann zur Förderung des Gases eingesetzt werden, da während der Schwitz- und einem Teil der Abschmelzphase keine Schmelze in der Zirklulationsleitung 21 zirkuliert wird und diese somit leer ist. Die erfindungsgemässe Verbesserung kann mit geringem Aufwand bei bestehenden Kristallisatoren zur Leistungssteigerung nachgerüstet werden.

Die Verbindungsleitung 31 ist durch Ventile 37,39 absperrbar. Ein Gebläse 41 dient der Förderung des in der Leitung 31 befindlichen Gases, z.B. Luft. Das Gas wird durch einen in der Verbindungsleitung 31 angeordneten Wärmetauscher 43 erwärmt. Der Wärmetauscher 43 kann eine Wärmetauscherfläche, eine elektrische Heizschlange, ein Ofen, welcher um die Leitung 31 herum angeordnet ist, oder dergleichen sein. Um das Gas möglichst rasch auf die gewünschte Temperatur erwärmen zu können, ist der Wärmetauscher 43 vorzugsweise in die Verbindungsleitung 31 integriert. Vorzugsweise besteht der Wärmetauscher 43 aus Warmetauscherrohren 45, welche durch ein Wärmetransfermedium, z.B. heisser Wasserdampf, durchströmbar sind. Ein Regulierventil 49, ein Temperaturfühler 51 und ein Steuergerät 53 bilden zusammen einen Regelkreis, welcher die Einhaltung von definierten und reproduzierbaren Bedingungen über einen grösseren Zeitraum ermöglichen.

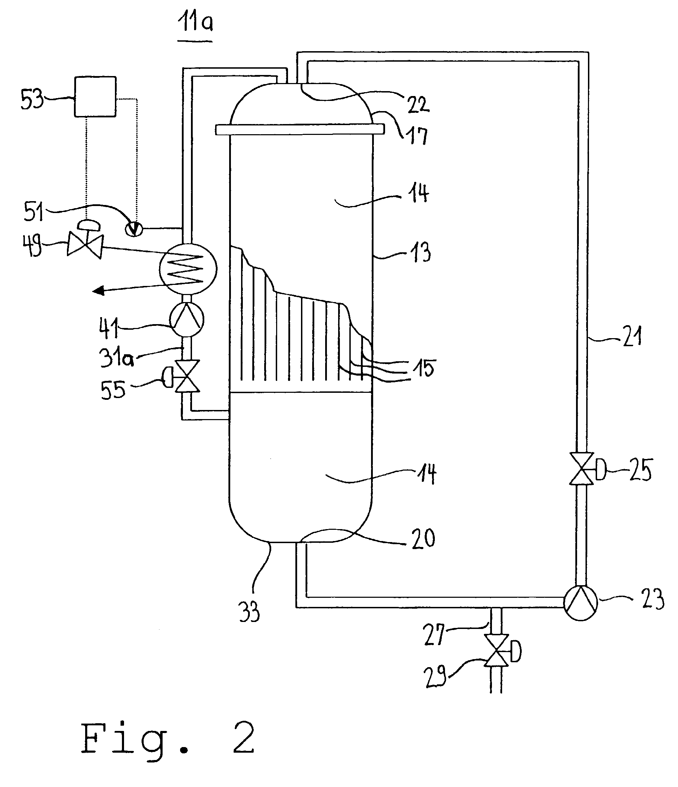

Der Kristallisator 11a gemäss Figur 2 unterscheidet sich von demjenigen von Figur 1 darin, dass die Verbindungsleitung 31a nicht in die Flüssigkeitszirkulationsleitung sondern direkt in den Kristallisatordeckel mündet. Die Verbindungsleitung 31a ist durch ein mit der Referenznummer 55 bezeichneten Absperrventil absperrbar.

Grundsätzlich denkbar ist, das Gas nur einmal zu gebrauchen und entsprechend nicht im Kreislauf zu führen, sondern in die Umgebung abzulassen.

Im Falle eines statischen Kristallisators erstrecken sich die Wärmetauscherflächen im wesentlichen über die ganze Höhe des Kristallisators. Entsprechend sind für die Gaszirkulation der Gasauslass näher zum Boden zu wählen als bei einem Fallfilmkristallisator. Grundsätzlich denkbar ist auch, die Vorrichtungen, Leitungen etc. wenigstens teilweise im Kristallisator zu führen.

Die Reinigung eines Verbindungsgemisches mittels Schichtkristallisation, z.B. in einem Fallfilmkristallisator, geschieht folgendermassen: Ein zu reinigendes, flüssiges Verbindungsgemisch wird in den Kristallisator eingebracht, wobei die eingebrachte Menge bis knapp an die Wärmetauscherflächen reichen kann. Danach wird das flüssige Verbindungsgemisch in der Leitung 21 zirkuliert, und die Temperatur der Wärmetauscherflächen 15 abgesenkt, indem das in diesen zirkulierende Wärmetauschermedium mittels eines Wärmetauschers gekühlt wird. Sobald die Wärmetauscherflächen 15 eine bestimmte Temperatur unterhalb der Schmelztemperatur der gewünschten Verbindung erreicht, beginnt diese auszukristallisieren und auf den Wärmetauscherflächen als Kristallschicht abzuscheiden. Wenn die Kristallschicht eine bestimmte Stärke erreicht hat, wird der Kristallisationsvorgang abgebrochen und die nicht-kristallisierte Mutterlauge in einen Tank abgelassen. Danach werden die Wärmetauscherflächen langsam erwärmt, um die Kristalle zum Schwitzen zu bringen. Gleichzeitig wird ein erwärmter Gasstrom über die Kristallschicht geblasen, sodass die Kristallschicht von zwei Seiten erwärmt wird. Vorzugsweise wird der zuvor erwärmte Gasstrom von oben nach unten über die Kristallschicht geleitet. Dies hat den Vorteil, dass die Kristallschicht oben stärker angeschmolzen wird als unten, da der Gasstrom relativ rasch seinen Wärmeinhalt an die Umgebung abgibt und somit weiter unten im Kristallisator merklich kühler ist als beim Eintritt in den Kristallisator. Die durch das Kristallschwitzen erzeugte Schmelze fliesst über die noch feste Kristallschicht nach unten, wobei diese durch die abfliessende Schmelze gewaschen wird. Dadurch wird die Trennleistung verbessert. Die Schwitzphase wird im unteren Kristallisatorraum gesammelt, in einen Tank abgelassen und für eine nachfolgende Reinigung zwischengelagert.

Alternativ zur oben angegebenen Vorgehensweise kann zur Reinigung von an den Wärmetauscherflächen schlecht haftenden Stoffen oder Stoffgemischen das in den Wärmetauschern zirkulierende Wärmetauschermedium auf einer Temperatur unterhalb der Kristallisationstemperatur der Schicht belassen werden und das Schwitzen ausschliesslich durch einen erwärmten Gasstrom erfolgen. Dies hat den Vorteil, dass während des Schwitzens für eine gute Haftung der Kristallschicht an den Kristallisatorflächen gesorgt ist.

Nach dem Schwitzen wird die verbleibende Kristallschicht abgeschmolzen und gegebenfalls neuerlich einem Reinigungszyklus unterzogen. Fraktionen verschiedener Reinigungszyklen mit ungefähr gleicher Zusammensetzung können zusammengelegt und gemeinsam einem Kristallisations/Schmelzvorgang unterworfen werden.

Das Kristallisationsverfahren in einem statischen Kristallisator unterscheidet sich vom oben beschriebenen darin, dass der Kristallisator soweit mit Schmelze gefüllt wird, dass die Wärmetauscherflächen durch die Schmelze bedeckt sind. Danach werden die Wärmetauscherflächen abgekühlt und das gewünschte Produkt als Kristallschicht abgeschieden, wobei während des Kristallisationsvorgangs praktisch keine Agitation des flüssigen Kristallisatorinhalts stattfindet. Das Schwitzen und Abschmelzen der Kristallschicht kann wie oben mit Unterstützung eines erwärmten Gasstromes erfolgen, wobei auch beim statischen Kristallisationsverfahren eine Verbesserung der Trennleistung erreichbar ist.

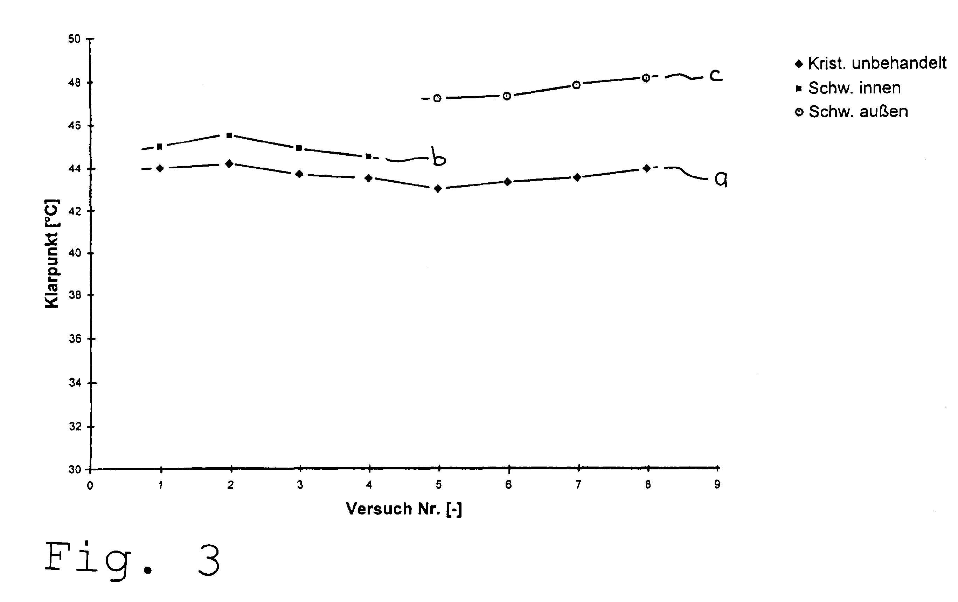

Figur 3 zeigt anhand einer vergleichenden Darstellung die Verbesserung, die mit dem erfindungsgemässen Verfahren erreichbar ist.

Gereinigt wurde ein wasserfreies Butterfett (= Fettsäuregemisch). Verglichen wurde jeweils der Klarpunkt
a) der auf den Wärmetauscherflächen abgeschiedenen Kristallschicht (ohne Schwitzen Kurve a);
b) der Kristallschicht nach konventionellem Schwitzen (Erwärmung der abgeschiedenen Kristallschicht von innen, d.h. durch Erwärmung des Wärmetransfermediums, welches auf der der Kristallschicht gegenüberliegenden Seite der Wärmetauscherflächen vorbei geleitet wurde (Kurve b); und
c) der Kristallschicht nach erfindungsgemässem Schwitzen von aussen, d.h. durch Darüberleiten eines erwärmten Gasstromes (Luft) über die Kristallschichten (Kurve c).

Es wurden insgesamt 8 Proben von Butterfett kristallisert und die erzielte Verbesserung der Eigenschaft der Proben, insbesondere Erhöhung des Schmelzpunktes, wurde anhand des Klarpunktes der abgeschiedenen Kristallschicht festgestellt. Es ist bekannt, dass ein höherer Klarpunkt mit einer günstigeren Fettsäurezusammensetzung korreliert. Zum Gasschwitzen wurde Luft eingesetzt.

Das eingesetzte wasserfreie Butterfett besass vor der Reinigung einen Klarpunkt von 36 °C. Durch die eingesetzte dynamische Kristallisation mittels Fallfilm erhöhte sich der Klarpunkt des Butterfetts auf ca. 43 bis 44 °C (Kurve a).

Anschliessend wurden die Proben 1 bis 4 konventionell geschwitzt. Zum (konventionellen) Schwitzen, d.h. Erwärmung der abgeschiedenen Kristallschicht durch das in den Wärmetauscherflächen zirkulierende Wärmetransfermedium, wurde das Wärmetransfermedium während 20 Minuten auf einer Temperatur von 35 °C belassen. Ein längeres Schwitzen wurde durch die geringe Haftung der Kristallschicht an den Wärmetauscherflächen verhindert (Gefahr des Abrutschens der Schicht). Nach dem Schwitzen wurde der Klarpunkt der Kristallschicht neuerlich gemessen (Kurve b).

Der Vergleich der Kurve a (Versuche 1 bis 4) mit der Kurve b zeigt, dass durch das bekannte Schwitzverfahren von innen (d.h. via der Wärmetauscherflächen) der Klarpunkt um ca. 1.0 bis 1.2 °C erhöht werden konnte.

Zum Gasschwitzen wurde auf ca. 80 °C erwärmte Luft über die Kristallschicht geleitet (Versuche 5 bis 8). Während dieses Vorgangs wurde die Temperatur des flüssigen Wärmetransfermediums auf ca. 15 °C gehalten, sodass die Kristallschicht an den Wärmetauscherflächen gut haftete und ein Abrutschen verhindert wurde. Das Gasschwitzen wurde eingestellt, sobald praktisch keine Schmelze von der Kristallschicht mehr abtropfte (nach ca. 1 Stunde).

Der Vergleich der Kurve a (Versuche 5 bis 8) mit der Kurve c (Gasschwitzen) zeigt, dass durch das erfindungsgemässe, von aussen durch eine Gasphase induzierte Schwitzverfahren der Klarpunkt um ca. 4 °C erhöht werden konnte. Dieses im Vergleich zum bekannten Schwitzverfahren wesentlich bessere Ergebnis war überraschend und unerwartet.

Vorteilhaft eignet sich das neue Verfahren zur Reinigung von Acrylsäure, Metacrylsäure, Abwasser, Methyldiphenylisozyanat (MDI), Toluoldiisozyanat (TDI), Caprolactam, Benzoesäure, Bisphenol-A-, Nitrochlorbenzol, geradkettige und verzweigte Fettsäure, Hydrazin, Phenole wie para-, meta und ortho-Kresol, 2.6 und 3,5-Dimethylphenol, Naphthol und o,o-Diphenol, chlorierte Kohlenwasserstoffe wie Dichlorbenzol und Nitrochlorbenzol, Naphthalin, 1- 2-Methylnaphthalin, Acenaphthen, Fluoren, Phenanthren, Adipinsäuredinitril, Hexamethylendiamin, Lactide (Polylactide), Chlorpyrifos, Fettsäuregemische, Butterfett sowie Paraffine ab C₁₇.

Das erfindungsgemässe Verfahren lässt sich bei allen Kristallisatortypen ( z.B. vom Typ Fallfilm, volldurchströmtes Rohr, Kristallisator mit flachen, z.B. doppelwandigen, Wärmetauscherflächen) einsetzen, welche ein dynamisches oder ein statischen Kristallisationsverfahren anwenden und die zu reinigende Verbindung als Kristallschicht auf Wärmetauscherflächen abgeschieden wird. Bestehende Kristallisatoren können mit geringem Aufwand nachgerüstet werden, um die Anwendung des erfindungsgemässen Gasschwitzens zu ermöglichen.

## Patentansprüche

1. Verfahren zur Trennung oder Reinigung von gelösten, geschmolzenen oder flüssigen Verbindungen oder Verbindungsgemischen mittels fraktionierter Kristallisation, bei welchem Verfahren
a) ein zu reinigende Verbindung oder zu trennendes Verbindungsgemisch in einen Behälter (13) eingebracht und in Kontakt mit Wärmetauscherflächen (15) gebracht wird,
b) die Wärmetauscherflächen (15) auf der einen Seite mittels eines Wärmetransfermediums von einer Temperatur, welche vorzugsweise höher ist als jene, bei welcher die gewünschte Verbindung kristallisiert, auf eine Endtemperatur, bei welcher die gewünschte Verbindung wenigstens teilweise kristallisiert, abgekühlt werden, sodass sich eine aus Kristallen bestehende Kristallschicht der gewünschten Verbindung auf der anderen Seite der Wärmetauscherflächen bildet,
c) dann der nicht-kristallisierte Teil der Verbindung oder des Verbindungsgemisches abgelassen wird und
d) die Kristallschicht geschmolzen und die Schmelze in wenigstens einer Fraktion gesammelt und gegebenfalls, falls der gewünschte Reinheitsgrad noch nicht erreicht ist, einem neuen Reinigungszyklus mit den Schritten a) bis d) unterworfen wird,
**dadurch gekennzeichnet, dass**
e) dass zum wenigstens teilweise Schmelzen der gebildeten Kristallschicht wenigstens zeitweise ein Gasstrom eines gasförmigen Wärmetransfermediums über die Kristallschicht geleitet wird, wobei der Gasstrom vorgängig auf eine Temperatur erwärmt wird, welche höher als die Schmelztemperatur der Kristallschicht ist.

2. Verfahren nach Anspruch 1, dadurch gekennzeichnet, dass das Schmelzen geschieht, indem die Kristallschicht zuerst geschwitzt und dann abgeschmolzen wird, wobei wenigstens zum Schwitzen wenigstens zeitweise ein Gasstrom eines gasförmigen Wärmetransfermediums über die Kristallschicht geleitet wird.

3. Verfahren nach Anspruch 2, dadurch gekennzeichnet, dass zum Schwitzen einerseits die Temperatur des Wärmetransfermediums erhöht und andererseits wenigstens zeitweise ein erwärmter Gasstrom eines gasförmigen Wärmetransfermediums über die Kristallschicht geleitet wird.

4. Verfahren nach Anspruch 2, dadurch gekennzeichnet, dass zum Schwitzen die Temperatur des Wärmetransfermediums auf einer Temperatur, welche tiefer ist als der Schmelzpunkt der Kristallschicht, belassen wird und wenigstens zeitweise ein erwärmter Gasstrom eines gasförmigen Wärmetransfermediums über die Kristallschicht geleitet wird.

5. Verfahren nach einem der Ansprüche 1 bis 4, dadurch gekennzeichnet, dass ein Inertgas, z. B. Edelgas, Kohlendioxid, Stickstoff, über die Kristallschicht geleitet wird.

6. Verfahren nach einem der Ansprüche 1 bis 4, dadurch gekennzeichnet, dass eine nicht-reaktive Kohlenwasserstoffverbindung oder ein Gemisch von nicht-reaktiven Kohlenwasserstoffverbindungen über die Kristallschicht geleitet wird.

7. Verfahren nach einem der Ansprüche 1 bis 6, dadurch gekennzeichnet, dass der Gasstrom in Gleichrichtung zur abfliessenden Schmelze über die Kristallschicht geleitet wird.

8. Verfahren nach einem der Ansprüche 1 bis 7, dadurch gekennzeichnet, dass der Gasstrom im Kreis geführt wird.

9. Verfahren nach einem der Ansprüche 1 bis 8, dadurch gekennzeichnet, dass der Gasstrom auf eine Temperatur erhitzt wird, welche wenigstens 5 °C, vorzugsweise wenigstens 15 °C und ganz besonders bevorzugt wenigstens 25 °C über dem Schmelzpunkt der die Kristallschicht bildenden Kristalle liegt.

10. Verfahren nach einem der Ansprüche 1 bis 9, dadurch gekennzeichnet, dass die Erwärmung des Gasstromes geregelt erfolgt.

11. Kristallisator (11;11a) mit
a) einem Behälter (13) mit einem Behälterraum (14) zur Aufnahme eines zu trennenden Verbindungsgemisches oder einer zu reinigenden Verbindung
b) Wärmetauscherflächen, welche in Verbindung mit dem Behälterraum (14) stehen,
c) wenigstens einem Einlass im Kopfbereich (17) des Kristallisators (11;11a) zur Einleitung eines im wesentlichen flüssigen oder geschmolzenen Verbindungsgemisches in den Behälterraum (14) und
d) wenigstens einem Auslass im Bodenbereich des Kristallisators zur Ableitung des im wesentlichen flüssigen Verbindungsgemisches aus dem Behälterraum (14),
e) einer Zirkulationsleitung (21), welche den Auslass mit dem Einlass verbindet,
f) wenigstens einer Pumpe (23), welche mit der Zirkulationsleitung (21) in Verbindung steht, zur Förderung des Verbindungsgemischs vom Bodenbereich (19) zum Kopfbereich (17) des Kristallisators, und
g) Mitteln (27,29) zum Zuführen von frischem Material in den Kristallisator (11;11a) resp. Ableitung von gereinigtem Material aus dem Kristallisator (11;11a), **gekennzeichnet durch,**
h) wenigstens einen Gaseinlass für den Einlass eines Gases in den Behälterraum (14) und
i) wenigstens einen Gasauslass für den Auslass des gasförmigen Behälterinhalts aus dem Behälterraum (14),
k) einer Gaszuleitung mit Heizmitteln (43), welche mit dem Gaseinlass in Verbindung steht zur Erwärmung des zuzuführenden Gases, und
l) Fördermitteln (41) zur Förderung des Gases in resp. aus dem Behälterraum (14).

12. Kristallisator nach Anspruch 12, dadurch gekennzeichnet, dass der Gasein- und der Gasauslass durch eine Verbindungsleitung (31;31a) miteinander verbunden sind.

13. Kristallisator nach Anspruch 12 oder 13, dadurch gekennzeichnet, dass die Verbindungsleitung (31) teilweise durch die Zirkulationsleitung (21) gebildet ist.

14. Kristallisator nach einem der Ansprüche 12 bis 14, dadurch gekennzeichnet, dass der Gaseinlass in Abstand zum Kristallisatorboden (33) vorgesehen ist.

15. Kristallisator nach einem der Ansprüche 12 bis 15, dadurch gekennzeichnet, dass die Verbindungsleitung mittels Absperrmitteln (37,39) absperrbar ist.

16. Kristallisator nach einem der Ansprüche 12 bis 16, dadurch gekennzeichnet, dass die Heizmittel (43) durch einen Wärmetauscher, z.B. eine Heizschlange (45), in welchem ein Wärmetauschermedium zirkulieren kann, gebildet sind.

17. Kristallisator nach einem der Ansprüche 12 bis 17, dadurch gekennzeichnet, dass die Heizmittel (43) mit einem Temperaturfühler (51) und einem Regulierventil (49), welches den Durchfluss des Wärmetauschermediums im Wärmetauscher kontrolliert, in Verbindung stehen.

18. Kristallisator nach einem der Ansprüche 12 bis 18, dadurch gekennzeichnet, dass zur Bildung eines Regelkreises der Temperaturfühler (51) und das Regulierventil (49) mit einem Steuergerät (53) in Verbindung stehen.

19. Kristallisator nach einem der Ansprüche 12 bis 19, dadurch gekennzeichnet, dass die Fördermitteln (41) derart ausgebildet sind, dass im Betrieb das Gas vom Boden- (19) zum Kopfbereich (17) des Kristallisators gefördert wird.

20. Kristallisator nach einem der Ansprüche 12 bis 20, dadurch gekennzeichnet, dass der Kristallisator vom Typ Fallfilmkristallisator, Kristallisator mit voller Rohrfüllung oder statischer Kristallisator ist.

21. Verwendung eines Verfahrens gemäss einem der Ansprüche 1 bis 11 resp. eines Kristallisators gemäss einem der Ansprüche 12 bis 20 zur Reinigung von Acrylsäure, Metacrylsäure, Abwasser, Methyldiphenylisozyanat (MDI), Toluoldiisozyanat (TDI), Caprolactam, Benzoesäure, Bisphenol-A-, Nitrochlorbenzol, geradkettige und verzweigte Fettsäure, Hydrazin, Phenole wie para-, meta und ortho-Kresol, 2.6 und 3,5-Dimethylphenol, Naphthol und o,o-Diphenol, chlorierte Kohlenwasserstoffe wie Dichlorbenzol und Nitrochlorbenzol, Naphthalin, 1- 2-Methylnaphthalin, Acenaphthen, Fluoren, Phenanthren, Adipinsäuredinitril, Hexamethylendiamin, Lactide (Polylactide), Chlorpyrifos, Fettsäuregemische, Butterfett sowie Paraffine ab C₁₇.
